# EUROPEAN PATENT SPECIFICATION

(11) **EP 1 993 363 B1**
(45) Date of publication and mention of the grant of the patent: **14.07.2010**
(21) Application number: 06762628.3
(22) Date of filing: 17.07.2006
(51) Int. Cl.: A01N 55/10, A01P 7/00, A01P 15/00, A01N 25/30

(54) **COMPOSITION FOR COMBATING ECTOPARASITES AND THEIR OVA**
ZUSAMMENSETZUNG ZUR BEKÄMPFUNG VON EKTOPARASITEN UND IHREN EIERN
COMPOSITION DESTINEE A LA LUTTE CONTRE LES ECTOPARASITES ET LEURS OVULES

(30) Priority: 13.03.2006 DE 202006004172 U
(43) Date of publication of application: 26.11.2008
(73) Proprietor: G. Pohl-Boskamp GmbH & Co. KG, 25551 Hohenlockstedt (DE)
(72) Inventor: BOSKAMP, Marianne, 25524 Bekmünde (DE); HILSCHER, Daniel, 24145 Kiel (DE); VATER, Thomas, 22299 Hamburg (DE)
(74) Representative: Hamm, Volker
(86) International application number: PCT/EP2006/006988
(87) International publication number: WO 2007/104345

(56) References cited:
- EP-A- 0 596 304
- EP-B- 1 215 965
- DE-A1- 2 823 595
- US-A1- 2003 202 997

## Description

The present invention pertains to compositions for combating ectoparasites, in particular head lice, as well as their eggs (nits).

Ectoparasites, such as lice, are parasites of the skin which are preferably located in very hairy regions. There, the female species lay ova which are encased in protective sheaths adhered to the hairs; in the case of lice these ova are also referred to as nits. Eggs of ectoparasites are often encased in protective sheaths which cannot be attacked by many toxic agents.

Neither lice nor nits can be removed by a normal hair wash, only a local treatment with suited agents, in particular insecticides, can be successful to remove lice and/or nits. In Germany, there are currently six drug-products for treating head lice on the market which contain as active ingredients allethrin, lindan, permethrin and pyrethrum, respectively. These active ingredients are very efficient, however, they are of toxicological concern and recently the development of resistances against these agents has been observed.

Various cosmetic and medicinal products for controlling ectoparasites have been developed as alternatives to drug-products, however, many of these products proved to be less or non efficient. A promising approach is the use of siloxane polymers for combating ectoparasites. For instance, the German patent application DE 2 823 595 discloses compositions for controlling lice and/or their eggs, comprising an amount of linear siloxane polymers, in particular dimethicones. It is explicitly stated in said patent application that dimethicones having a viscosity in the range from 100 to 1000 cSt are most efficient in combating lice.

European patent application EP 1 215 965 discloses compositions for combating arthropodes comprising two different silicones, one of which is a volatile siloxane polymer and the other is a non-volatile siloxane polymer. The volatile siloxane is preferably a cyclic siloxane such as cyclopentasiloxane or cyclomethicone, which is used in amounts of 95.5-97.5 wt.-%, based on the composition.

While some of these prior art compositions are satisfactory in combating lice, they have a very low ovicidal efficacy, i.e. they do not destroy nits reliably. A further disadvantage of these known compositions is that they are potentially noxious.

Thus, there is still a need for toxicologically acceptable but highly effective compositions for combating ectoparasites and their eggs.

In experiments underlying the present invention it was surprisingly found that compositions comprising a very low viscosity linear polysiloxane, a higher viscosity linear polysiloxane, and a spreading agent are highly effective against lice as well as against nits. Due to the omission of cyclic siloxanes these compositions are further very well tolerated.

The compositions according to the invention have a very low surface tension and, at the same time, a high spreadability. Without wishing to be bound to any theory, it is believed that the compositions due to their very favourable creep properties can penetrate deeply into the tracheae and tracheols of the adult head lice and their larvas, and into the aeropyles of the nits. There, the volatile siloxane used in accordance with the invention evaporates, whereupon the composition in the trachea and the nits, respectively, becomes more and more thick and eventually clots. As a consequence, the lice, larvae and nits are separated from a gas exchange and suffocate.

The compositions according to the invention comprise a linear polysiloxane having a viscosity < 10 cSt and a linear siloxane having a viscosity > 90 cSt. Dimethicones are preferred polysiloxanes. According to the preferred embodiment the composition comprises dimethicone having a viscosity of 1 cSt and dimethicone having a viscosity of 100 cSt. The low viscosity polysiloxane is contained in amounts of from 30-49 wt.-% and the higher viscosity polysiloxane is contained in amounts of from 35-65 wt.-%, in each case based on the total weight of the composition.

The compositions according to the invention further comprise a spreading agent such as well spreading triglycerides derived from short chain or medium chain fatty acids such as medium chain triglycerides according to the European Pharmacopeia, coconut oil, palm kernel oil, babussu oil, glycerol triacetate, or well-spreading waxes or esters of fatty acids and fatty alcohols such as jojoba wax, cetearyl isononanoate, cetearyl octanoate, isopropylmyristate, isopropylpalmitate, ethylhexylpalmitate or cocoyl caprylocaprate. Preferably, the compositions according to the invention contain 2-10 wt.-% medium chain triglycerides and/or 1-5 wt.-% jojoba wax.

The compositions can further comprise common cosmetic excipients such as fragrances or common skin care components.

The following examples illustrate the invention in more detail.

### Examples

Compositions comprising the ingredients listed in the following table were produced:

| Ingredient | Inv. 1 | Inv. 2 | Comp. |
|---|---|---|---|
| Dimethicone 1 cSt | 40 | 42 | 92 |
| Dimethicone 100 cSt | 50 | 50 | - |
| Medium chain triglycerides | - | 5 | 5 |
| Jojoba wax | 2 | 2 | 2 |
| Fragrance | 1 | 1 | 1 |
| Babussu oil | 5 | - | - |
| Isododecane | 2 | - | - |

All amounts are indicated in wt.-%.

The compositions "Inv. 1" and "Inv. 2" according to the invention and the comparative composition "Comp." were examined with respect to their efficacy in killing lice and their ova. For this purpose the compositions were left on stage 3 lice larvae (which are indicated as reference for motile lice stages (adult lice and larvae)), on nits being younger than 24 hours ("Nits I"), and on nits being older than 24 hours ("Nits II") for 45 minutes. The results of these experiments are summarized in the following table, whereby the percentages relate to the amount of killed larvae/nits in the respective stages.

| Composition | Larvae | Nits I | Nits II |
|---|---|---|---|
| Inv. 1 | 100 % | 75,7 % | 39,5 % |
| Inv. 2 | 100 % | 100 % | 67,5 % |
| Comp. | 100 % | 27,5 % | 29,1 % |

It can be seen that all compositions have a very high efficacy in killing lice, but that only the combination of a high viscosity polysiloxane, a low viscosity polysiloxanes and a spreading agent in accordance with the invention exhibits a satisfactory ovicidal efficacy.

## Claims

1. Composition for killing ectoparasites and/or their ova, comprising 30-49 wt.-%, based on the composition, of a low viscosity linear polysiloxane having a viscosity < 10 cSt, 35-65 wt.-%, based on the composition, of a higher viscosity linear polysiloxane having a viscosity > 90 cSt, and at least one spreading agent.

2. Composition according to claim 1, **characterized in that** the spreading agent is selected from medium chain triglycerides, coconut oil, palm kernel oil, babussu oil, glycerol triacetate, jojoba wax, cetearyl isononanoate, cetearyl octanoate, isopropylmyristate, isopropylplamitate, ethylhexylpalmitate and cocoyl caprylocaprate.

3. Composition according to claim 1 or claim 2, **characterized in that** it contains 2-10 wt.-% of medium chain triglycerides and/or 1-5 wt.-% jojoba wax as spreading agent, the percentages being based on the composition.

4. Composition according to any of claims 1-3, **characterized in that** the low viscosity polysiloxane is dimethicone having a viscosity of 1 cSt and the higher viscosity polysiloxane is dimethicone having a viscosity of 100 cSt.

## Patentansprüche

1. Zusammensetzung zur Abtötung von Ectoparasiten und / oder deren Eiern, umfassend 30-49 Gew.-%, bezogen auf die Zusammensetzung, eines niedrigviskosen linearen Polysiloxans mit einer Viskosität < 10 cSt, 35-65 Gew.-%, bezogen auf die Zusammensetzung, eines höherviskosen linearen Polysiloxans mit einer Viskosität > 90 cSt sowie mindestens ein Spreitmittel.

2. Zusammensetzung nach Anspruch 1, **dadurch gekennzeichnet, dass** das Spreitmittel ausgewählt ist aus mittelkettigen Triglyceriden, Kokosöl, Palmkernöl, Babussuöl, Glyceroltriacetat, Jojobawachs, Cetearylisononanoat, Cetearyloctanoat, Isopropylmyristat, Isopropylpalmitat, Ethylhexylpalmitat und Cocoylcaprylocaprat.

3. Zusammensetzung nach Anspruch 1 oder Anspruch 2, **dadurch gekennzeichnet, dass** als Spreitmittel 2-10 Gew.-% mittelkettige Triglyceride und/oder 1-5 Gew.-% Jojobawachs, jeweils bezogen auf die Zusammensetzung, enthalten sind.

4. Zusammensetzung nach einem der Ansprüche 1-3, **dadurch gekennzeichnet, dass** das niedrigviskose Polysiloxan Dimeticon mit einer Viskosität von 1 cSt und das höherviskose Polysiloxan Dimeticon mit einer Viskosität von 100 cSt ist.

## Revendications

1. Composition destinée à tuer des ectoparasites et/ou leurs ovules, comprenant 30 à 49 % en poids, par rapport à la composition, d'un polysiloxane linéaire à faible viscosité ayant une viscosité < 10 cSt, 35 à 35 % en poids, par rapport à la composition, d'un polysiloxane linéaire à viscosité élevée ayant une viscosité > 90 cSt, et au moins un agent de dispersion.

2. Composition selon la revendication 1, **caractérisée en ce que** l'agent de dispersion est sélectionné parmi des triglycérides à chaîne moyenne, l'huile de noix de coco, l'huile de palmiste, l'huile de babassu, le triacétate de glycérol, la cire de jojoba, l'isononanoate de cétéaryle, l'octanoate de cétéaryle, le myristate d'isopropyle, le palmitate d'isopropyle, le palmitate d'éthylhexyle et le caprylocaprate de cocoyle.

3. Composition selon la revendication 1 ou 2, **caractérisée en ce qu'**elle contient 2 à 10 % en poids de triglycérides à chaîne moyenne et/ou 1 à 5 % en poids de cire de jojoba en tant qu'agent dispersion, les pourcentages étant basés sur la composition.

4. Composition selon l'une quelconque des revendications 1 à 3, **caractérisée en ce que** le polysiloxane à faible viscosité est du diméthicone ayant une viscosité de 1 cSt et le polysiloxane à viscosité élevée est du diméthicone ayant une viscosité de 100 cSt.
